# EUROPÄISCHE PATENTANMELDUNG

(11) **EP 1 530 975 A1**
(43) Veröffentlichungstag der Anmeldung: **18.05.2005**
(21) Anmeldenummer: 04026508.4
(22) Anmeldetag: 09.11.2004
(51) Int. Cl.: A61L 9/03

(54) **Vorrichtung zur Abgabe von Duftstoffen**

(30) Priorität: 14.11.2003 DE 10353286
(71) Anmelder: IFG Institut für Geruchsforschung AG, 8853 Lachen (CH)
(72) Erfinder: Voit, Hans, 82166 Gräfelfing (DE)
(74) Vertreter: Haft, von Puttkamer, Berngruber, Karakatsanis

(57) **Zusammenfassung**

Eine Vorrichtung zur Abgabe von Duftstoffen weist einen Vorratsbehälter (1) für die Duftstofflösung und eine mit einer Heizeinrichtung erwärmbare, nach oben offene Verdampfungsschale (12) auf, der die Duftstofflösung von dem Vorratsbehälter (1) zugeführt wird, und zwar mit einer elektrochemischen Gasentwicklungszelle (5), die die Duftstofflösung aus dem Vorratsbehälter (1) verdrängt.

## Beschreibung

Die Erfindung bezieht sich auf eine Vorrichtung zur Abgabe von Duftstoffen nach dem Oberbegriff des Anspruchs 1.

Derartige Vorrichtungen sind bekannt (DE 9308368 U1; EP 1033139 A1). Dabei ist in einem säulenförmigen Gehäuse über dem Vorratsbehälter die Verdampfungsschale und darüber ein Ventilator vorgesehen, der die Duftstoffe aus dem Gehäuse bläst. Nach DE 9308368 U1 wird die Duftstofflösung mit einer Schlauchpumpe zur Verdampfungsschale gepumpt und nach EP 1033139 A1 ist als Pumpe eine Schraubenspindel vorgesehen, die in einem Rohr umläuft, das sich vom Vorratsbehälter zur Verdampfungsschale erstreckt.

Die bekannten Vorrichtungen haben sich zwar im Großen und Ganzen bewährt. Gleichwohl können sie noch verbessert werden.

So wird bei den bekannten Vorrichtungen mit der Pumpe eine bestimmte Menge Duftstofflösung in die Verdampfungsschale dosiert und die Pumpe dann abgeschaltet. Der intermittierende Pumpenbetrieb hat zur Folge, dass eine ungleichmäßige Duftabgabe erfolgt, da sich die Menge der Duftstofflösung in der Verdampfungsschale ständig ändert.

Zudem erfordern die Schläuche, Rohre usw., mit denen die Duftstofflösung der Verdampfungsschale zugeführt wird, eine regelmäßige sorgfältige Reinigung, um eine Verstopfung zu verhindern. Eine solche Reinigung ist jedoch vor allem auch dann erforderlich, wenn die Duftrichtung geändert und damit die Duftstofflösung ausgetauscht werden soll.

Aufgabe der Erfindung ist es, eine einfach bedienbare Vorrichtung bereitzustellen, die eine kontinuierliche Abgabe der gleichen Duftstoffmenge ermöglicht.

Dies wird erfindungsgemäß mit der im Anspruch 1 gekennzeichneten Vorrichtung erreicht. In den Unteransprüchen sind vorteilhafte Ausgestaltungen der Erfindung wiedergegeben.

Nach der Erfindung wird zur Zufuhr der Duftstofflösung von dem Vorratsbehälter zu der Verdampfungsschale eine elektrochemische Gasentwicklungszelle verwendet. Durch das von der Zelle entwickelte Gas wird die Duftstofflösung aus dem Vorratsbehälter verdrängt, so dass sie über die Austrittsöffnung des Vorratsbehälters in die Verdampfungsschale strömt. Die Verdampfungsschale ist dazu vorzugsweise mit einer elektrischen Heizung erwärmbar ausgebildet. Ferner kann zur Verbreitung des Duftes über der Verdampfungsschale ein Ventilator vorgesehen sein.

Somit wird eine kontinuierliche konstante Menge der Duftstofflösung der Verdampfungsschale zugeführt, so dass eine konstante gleichbleibende Menge des Duftstoffs abgegeben wird.

Die elektrochemische Gasentwicklungszelle wird vorzugsweise durch eine Batterie, einen Akku oder dergleichen netzunabhängige Energiequelle mit Strom versorgt. Sie besteht aus einer Elektrode, einer Gegenelektrode und einem Elektrolyt. Wenn ein wässeriger Elektrolyt verwendet wird, wird Wasserstoff- oder Sauerstoffgas entwickelt. Eine derartige Gasentwicklungszelle geht z.B. aus US 5,242,565 hervor. Die bekannte Gasentwicklungszelle wird für Schmiergeräte, zur Verabreichung von Medikamenten und als Referenzelektrode zur Messung der Wasserstoffionenkonzentration verwendet.

Nach einer Ausführungsform der erfindungsgemäßen Vorrichtung ist der Vorratsbehälter als Zylinder mit einem darin verschiebbaren Kolben ausgebildet. Während der Zylinderraum auf der Kolbenseite mit der Austrittsöffnung die Duftstofflösung aufnimmt, ist in dem anderen Zylinderraum die Gasentwicklungszelle angeordnet.

Der Zylinder ist dabei vorzugsweise aufrecht, also senkrecht angeordnet. Die Gasentwicklungszelle mit der netzunabhängigen Energieversorgung befindet sich am Boden des Zylinders. D.h., der untere Zylinderraum unter dem Kolben dient zur Aufnahme des von der Zelle gebildeten Gases. Der Zylinderraum über dem Kolben ist mit der Duftstofflösung gefüllt. Am oberen Ende des Zylinders befindet sich die Austrittsöffnung für die Duftstofflösung, vorzugsweise an einem Stutzen, der sich z.B. von der Mitte der oberen Stirnwand des Zylinders nach oben erstreckt.

Damit bildet der Zylinder mit dem Kolben, der Duftstofflösung und der Gasentwicklungszelle mit Energieversorgung eine Einheit, die lösbar entweder direkt oder über einen Adapter oder dergleichen an die Verdampfungsschale angeschlossen werden kann.

Zum lösbaren Anschluss kann beispielsweise der Stutzen an der oberen Zylinderwand eine Schraub-, Steck- oder dergleichen flüssigkeitsdichte Verbindung aufweisen, die über einen entsprechenden Anschluss mit der Verdampfungsschale verbunden werden kann.

Da die Einheit aus Zylinder, Kolben, Duftstofflösung, Gasentwicklungszelle und Energieversorgung nur ausgetauscht zu werden braucht, wenn die Duftstofflösung verbraucht ist oder ein anderer Duftstoff eingesetzt werden soll, ist die erfindungsgemäßen Vorrichtung außerordentlich leicht zu handhaben.

Auch ist es möglich, eine Verdampfungsschale zu verwenden, die mehrere Anschlüsse für mehrere dieser Einheiten mit unterschiedlichen Duftstofflösungen aufweist. Damit kann beispielsweise in einem Hotel zu Frühstück ein Orangenduft erzeugt werden und z.B. bei einer "italienischen Woche" der Duft von Zypressen und dergleichen südlichen Gewächsen usw.

Nach einer anderen Ausführungsform der Erfindung weist der Vorratsbehälter eine Steigleitung auf, die in die Duftstofflösung eintaucht und mit der Austrittsöffnung verbunden ist. Die Gasentwicklungszelle ist in dem Gasraum oberhalb der Duftstofflösung in dem Vorratsbehälter angeordnet, jedenfalls so, dass sie mit diesem Gasraum kommuniziert.

Um die Gasbildungsrate der Gasentwicklungszelle zu steuern, kann ein Stromregler vorgesehen sein. Auch ist es möglich, die Gasproduktion mit einem Timer zu unterbrechen.

Nachstehend ist die Erfindung anhand der beigefügten Zeichnung beispielhaft näher erläutert. Darin zeigen, jeweils schematisch:
- Figur 1: einen Längsschnitt durch eine erste Ausführungsform;
- Figur 2: einen Längsschnitt durch eine Variante der ersten Ausführungsform;
- Figur 3: einen Längsschnitt durch eine weitere Variante der ersten Ausführungsform; und
- Figur 4: einen Längsschnitt durch eine zweite Ausführungsform.

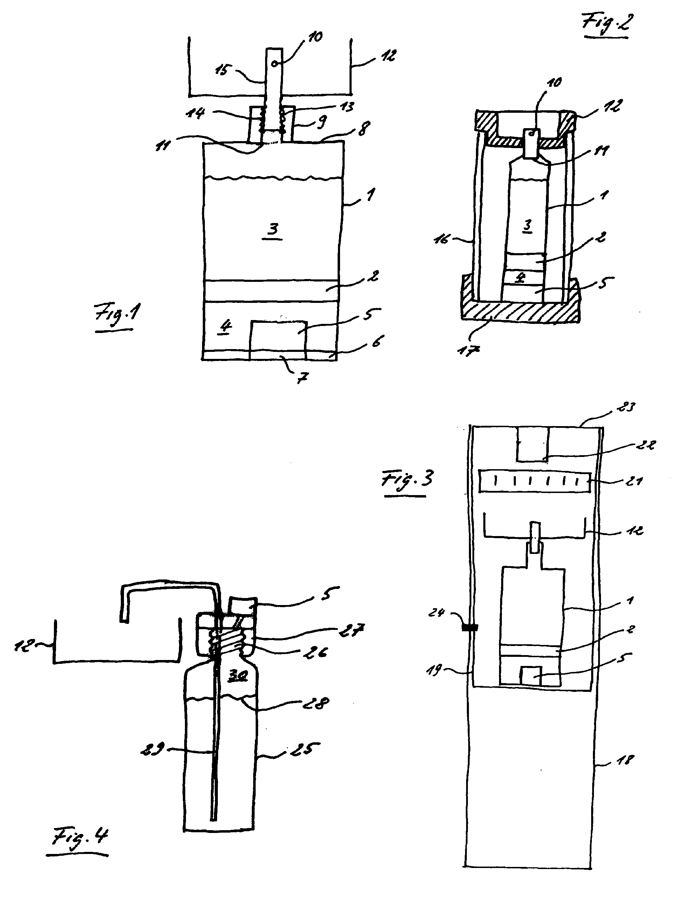

Die Vorrichtung nach Figur 1 besteht aus einem aufrecht stehenden Zylinder 1, in dem ein Kolben 2 verschiebbar geführt ist, der den oberen Zylinderraum 3, der mit der Duftstofflösung gefüllt ist, von dem unteren Zylinderraum 4 flüssigkeits- und gasdicht abdichtet.

In dem unteren Zylinderraum 4 ist in einem Gehäuse 5 die elektrochemische Gasentwicklungszelle mit Knopfbatterien zu deren Betrieb am Boden 6 vorgesehen. Mit dem Drehknopf 7, der im Zylinderboden 6 von außen zugänglich angebracht ist, wird die Stromzufuhr zur Gasentwicklungszelle und damit die Gasbildungsrate gesteuert.

An der oberen Stirnwand 8 des Zylinders 1 ist in der Mitte ein Stutzen 9 mit der Austrittsöffnung 11 für die Duftstofflösung im Zylinderraum 3 vorgesehen. Wenn die Duftstofflösung aus dem sonst hermetisch abgedichteten Zylinderraum 3 mit dem Kolben 2 verdrängt wird, tritt sie damit über die Austrittsöffnung 11 aus, um der nach oben offenen Verdampfungsschale 12 zugeführt zu werden. Zur Verbindung des Stutzens 9 mit der Verdampfungsschale 12 weist der Stutzen 9 ein Gewinde 13 auf, das an ein Gewinde 14 an einem Rohr 15 angeschraubt wird, das in die Mitte der Verdampfungsschale 12 ragt und an seinem über die Verdampfungsschale 12 hinausragenden oberen Ende oder am Umfang eine oder mehrere Öffnungen 10 aufweist, über die die Duftstofflösung in die Schale 12 fließt. Das Rohr 15 bildet damit die Duftstofflösungszufuhr zur Schale 12.

Bei der Ausführungsform nach Figur 2 ist der Zylinder 1 mit dem Kolben 2, der Duftstofflösung, dem Gehäuse 5 mit Gasentwicklungszelle und Energieversorgung in einem dazu konzentrisch angeordneten zylindrischen Außengehäuse 16 mit einem Boden 17 und der Verdampfungsschale 12 als Deckel angeordnet. Das Außengehäuse 16 kann aus einem durchsichtigen Material, beispielsweise Glas oder Acrylglas bestehen. Im Zylinderraum 3 kann eine farbige Duftstofflösung vorgesehen sein, wodurch die Vorrichtung nach Figur 2 ein ansprechendes Äußeres aufweist.

Gemäß Figur 3 weist die Vorrichtung als Außengehäuse 18 eine Säule auf, in der die Einheit aus Zylinder 1, Kolben 2, Duftstofflösung, Gehäuse 5 mit Gasentwicklungszelle und Energieversorgung in einem Einsatz 19 vorgesehen ist. Über der Verdampfungsschale 12 ist ein Ventilator 21 vorgesehen, ferner die Steuerung 22, mit der die Gasentwicklungszelle und die (nicht dargestellte) elektrische Heizung gesteuert wird, mit der die Verdampfungsschale 12 erwärmt wird, sowie gegebenenfalls die Drehzahl des Ventilators 21. Die Steuerung 22 ist über den Deckel 23 zugänglich, der die Säule 18 oben verschließt. Der Einsatz 19, einschließlich des Deckels 23, kann aus der Säule 18 oben herausgeschoben werden, um eine verbrauchte Einheit durch eine neue Einheit aus Zylinder 1, Kolben 2, Duftstofflösung, Gehäuse 5 mit Gasentwicklungszelle und Energieversorgung zu ersetzen. Der Einsatz 19 kann dazu mit einer (nicht dargestellten) Feder nach oben belastet und durch eine mit 24 schematisch angedeutete Verriegelungseinrichtung in der Säule 18 verriegelt sein.

Bei der Ausführungsform nach Figur 4 wird der Vorratsbehälter 25 durch eine Flasche mit einem Schraubgewinde 26 am Hals gebildet, auf das eine Kappe 27 aufgeschraubt wird, an der das Gehäuse 5 mit der Gasentwicklungszelle und der Batterie befestigt ist. Die Flasche 25 ist bis zu einem Niveau 28 mit der Duftstofflösung gefüllt. Ein Steigrohr 29 ragt durch die Kappe 27 zum Boden der Flasche 25. Das von der Gasentwicklungszelle im Gehäuse 5 gebildete Gas strömt über eine Bohrung 31 in der Kappe 27 in den Gasraum 30 über dem Niveau 28 der Duftstofflösung in der Flasche 25. Über das obere Ende des Steigrohres 29 fließt die Duftstofflösung in die Verdampfungsschale 12.

## Patentansprüche

1. Vorrichtung zur Abgabe von Duftstoffen mit einem Vorratsbehälter mit einer Austrittsöffnung für die Duftstofflösung und einer mit einer Heizeinrichtung erwärmbaren, nach oben offenen Verdampfungsschale, der die Duftstofflösung von dem Vorratsbehälter zugeführt wird, **dadurch gekennzeichnet, dass** zur Zufuhr der Duftstofflösung von dem Vorratsbehälter zur Verdampfungsschale (12) eine elektrochemische Gasentwicklungszelle vorgesehen ist, die die Duftstofflösung aus dem, bis auf die Austrittsöffnung (11) hermetisch abgedichteten Vorratsbehälter verdrängt.

2. Vorrichtung nach Anspruch 1, **dadurch gekennzeichnet, dass** der Vorratsbehälter als Zylinder (1) mit einem Kolben (2) ausgebildet ist, der Zylinderraum (3) auf der einen Kolbenseite mit der Austrittsöffnung (11) versehen ist und die Duftstofflösung aufnimmt, während der Zylinderraum (4) auf der anderen Kolbenseite das von der Gasentwicklungszelle entwickelte Gas aufnimmt.

3. Vorrichtung nach Anspruch 2, **dadurch gekennzeichnet, dass** der Zylinder (1) aufrecht angeordnet ist und der Zylinderraum (3) mit der Austrittsöffnung (11) über dem Kolben (2) die Duftstofflösung aufnimmt, während der Zylinderraum (4) unter dem Kolben (3) das von der Gasentwicklungszelle entwickelte Gas aufnimmt.

4. Vorrichtung nach Anspruch 3, **dadurch gekennzeichnet, dass** die Gasentwicklungszelle in dem unteren Zylinderraum (4) angeordnet ist.

5. Vorrichtung nach einem der Ansprüche 2 bis 4, **dadurch gekennzeichnet, dass** der Zylinder (1) mit der Duftstofflösung, dem Kolben (2) und der Gasentwicklungszelle mit der Energieversorgung eine Einheit zum lösbaren Anschließen an die Duftstofflösungszufuhr zur Verdampfungsschale (12) bildet.

6. Vorrichtung nach einem der Ansprüche 3 bis 5, **dadurch gekennzeichnet, dass** der Zylinder (1) mit dem Kolben (2), der Duftstofflösung und der Gasentwicklungszelle mit Energieversorgung in einem Außengehäuse (16, 18) angeordnet ist.

7. Vorrichtung nach Anspruch 6, **dadurch gekennzeichnet, dass** das Außengehäuse (16) aus einem durchsichtigen Material besteht.

8. Vorrichtung nach Anspruch 5 oder 6, **dadurch gekennzeichnet, dass** das Außengehäuse (18) als Säule ausgebildet ist, in der zum Austausch der Einheit aus Zylinder (1), Kolben (2), Duftstofflösung, Gasentwicklungszelle und Energieversorgung (5) ein Einsatz (19) vorgesehen ist.

9. Vorrichtung nach Anspruch 8, **dadurch gekennzeichnet, dass** der Einsatz (19) auch die Verdampfungsschale (12), einen Ventilator (21) sowie die Steuerung (22) der Vorrichtung aufnimmt.

10. Vorrichtung nach Anspruch 8 oder 9, **dadurch gekennzeichnet, dass** der Einsatz (19) aus der Säule (18)nach oben herhausbewegbar ausgebildet ist und auch den Deckel (23) aufweist, der die Säule (18) oben verschließt.

11. Vorrichtung nach Anspruch 1, **dadurch gekennzeichnet, dass** der Vorratsbehälter (25) ein Steigrohr (29) enthält und die Gasentwicklungszelle mit dem Gasraum (30) oberhalb der Duftstofflösung in dem Vorratsbehälter (25) kommuniziert.

12. Vorrichtung nach einem der vorstehenden Ansprüche, **dadurch gekennzeichnet, dass** die Stromzufuhr zur elektrochemischen Gasentwicklungszelle regelbar ausgebildet ist.
